# EUROPEAN PATENT APPLICATION

(11) **EP 1 913 868 A1**
(43) Date of publication of application: **23.04.2008**
(21) Application number: 06425721.5
(22) Date of filing: 19.10.2006
(51) Int. Cl.: A61B 5/055, G06T 7/00, G06F 19/00

(54) **System for determining diagnostic indications**

(71) Applicant: Esaote S.p.A., 20100 Milano (IT)
(72) Inventor: Biglieri, Eugenio, 15024 Masio (AL) (IT); Satragno, Luigi, 16122 Genova (IT)
(74) Representative: Karaghiosoff, Giorgio Alessandro

(57) **Abstract**

System for determining diagnostic indications which system comprises:
at least an apparatus for acquiring diagnostic images;
and image processing means for recognizing and measuring qualitative, quantitative, morphologic and/or dynamic characteristics of one or more objects reproduced in acquired images by pixels, or voxels or image data thereof

characterized in that
processing means and the apparatus for acquiring diagnostic images being integrated within the same device and the processing of the image or images being carried out directly at the end of the acquisition of the image or images as the final and/or intermediate step of the diagnostic imaging session or process.

## Description

The present invention relates to a system for determining diagnostic indications which system comprises:
at least an apparatus for acquiring diagnostic images;
and image processing means for recognizing and measuring qualitative, quantitative, morphologic and/or dynamic characteristics of one or more objects reproduced in acquired images by pixels, or voxels or image data thereof.

At present image processing means are known working on the basis of various algorithms or various "Image Processing" techniques and which are used in order to extract quantitative or qualitative information about conditions of structures reproduced in acquired images from image data or pixels or voxels of images, such as for example qualitative or quantitative indications about pathologic conditions of tissues, organs or fluids. These processing means are called aided diagnosys computer tools i.d. CAD since in parallel with the visual examination of images by the doctor, they provide an automatic or semi-automatic analysis of objective parameters that can be determined by acquired images constituting a criterion for indicating the probable presence or absence of specific diseases and/or specific evolution conditions of said diseases.

At present processing means having CAD functionalities are used by specialized personnel working in remote processing institutes and images and/or image data are sent thereto in order to be processed. This condition is an important limit, since even if personnel of processing institutes are certainly specialized in treating data, there is no possibility for a direct interaction with image acquisition process, therefore the image acquisition and processing of images by means having CAD functionalities remain two separate universes that communicate only as regards the mere data transmission.

Moreover generally various processing institutes are specialized in using some specific processing methods and generally the specialization in also limited in searching indications about some specific diseases.

Therefore there is no possibility in managing a plurality of different processing instruments each of which can be used in combination only with one or more further instruments in order to generate a sequence of processing processes optimizing the result or keeping the result reliability at a high level, when data of images have a poor quality or as it is said in jargon data are afflicted with a high noise.

For a considerable amount of diseases it has been found to be determining to have data indicating a certain probability about the presence or absence of the disease in very precocious conditions and in initial evolution conditions when functional damages are still limited and it is possible to carry out preventive therapeutic interventions slowing down or stopping the malignant evolution of the disease or bringing to the recovery.

From reserches made by various government or inter-government bodies, such as for example the world health organization some particular diseases have been identified whose impact on the population is important both as regards the numerical point of view and the social and personal damage point of view.

The rheumatic and orthopedic areas are among these ones. These are continuously increasing as regards the impact on the world population and they are one of the fields in which medical methods have most developped. Suffice it to mention the announcement of the initiative "Bone and Joint Decade: 2000-2010" made by the World Health Organization having the aim of improving the quality of life (from a medical point of view) of patients suffering from diseases regarding the muscle-skeletal field at a world level. Such diseases are one of the most common reason of invalidity and involve very high social and medical costs (the estimated value is about 215 billion dollars a year in the United States). Practically 90% of people sooner or later will suffer from painful diseases (the well-known backache) being the most important cause limiting working capacities of middle-aged people and one of the greatest cause for requiring medical examinations and generally health controls.

Another important area is cardio-circulatory diseases. These are the first cause of death with an impact of about 40% on all deadly events. Patients afflicted with said disease can meet the immediate death or can suffered from a cardiac infarction. The amount of people surviving a cardiac infarction has an expectation and quality of life that depends on various elements. Firstly it is important to immediately establish the size of the cardiac damage caused by the infarction in order to decide therapeutic measures for first intervention at the first request. Then once a rehabilitative and/or recovery therapy starts it is necessary to monitor the patient response. To this aim measures of cardiac function are necessary indicating the state of health of the damaged organ. Some of the current reference methods such as SPECT and high field nuclear magnetic resonance, lead to high examination costs and therefore to a relative spreading. Ultrasound diagnostics, provided with new and more developped tasks processing signals and images, can reach an equivalent informative level with lower costs.

The electronic technology supporting the ultrasound diagnostics has enormously increased during the last years and now it can provide information about the structure and function of the organ under examination with a good precision: three-dimensional geometry of the vessel inside, functional properties of the cardiac muscle, the geometry of a tumoral mass, or the vascularization of a tissue at tumoral risk, to mention some examples. Such information are used for the diagnosis of the presence and size of the disease and so to express a decision about needs and possible characteristics of the therapeutic treatment. However information detected by the electronic device are a measure of a very specific physical property related to the acquisition process i.e. the tissue reflexive property. The measured datum is then manipulated in order to be presented as images in order to make the visual interpretation easier by the clinic operator. Therefore diagnostic images reproduce a displaying of the detected datum. The provided representation is not the only possible displaying form, and often it is not the one direclty correlated with the analysed physiology or disease. Similarly the datum itself is the one directly acquired by the device and it is not necessarily a measure closely related to functional characteristics of the organ or it cannot be directly correlated to the actual physiopathologic situation.

There are various methods for acquiring diagnostic images and in some fields for example the use of the magnetic resonance is preferred and in other ones an ultrasound imaging method is preferred and still in other ones radiologic methods are used.

So called dedicated MRI systems known at present allow the diffusion of the resonance in studying diseases that, up to now, have been diagnosed mainly on the basis of clinical data. As regards all diagnostic images apart from the acquisition method, at present the diagnostic method that is used is the conventional radiology and i.e. the morphologic analysis of static images. This conventional analysis has proved its total inadequacy in finding many diseases in precocious stages that at present can be clinically treated by finding specific pharmacologic treatments, whose usefulness increases if they are begun upon the onset of the disease.

For example in the rheumatic field, the diagnosis by conventional radiology in based on highlithing bony erosions that are the effect of the disease after months or years of activity and represent the characteristic indication of a damage that is already occurred.

It is known that it is possible to have information about the potential presence of a disease in its precocious stages and so before the onset of the bony damage by examining the condition of the synovia and its possible inflammation and the presence of possible damages to the cartilage. To this aim the nuclear magnetic resonance examination is an ideal method just for the ability of highlithing synovial inflammatory conditions and possible cartilaginous damages, obviously besides allowing the detection of bony damages, all that with a panoramic view and a detail that are definitely higher than x-rays.

Despite these intrinsic abilities of the MRI diagnostic imaging and advantages deriving from using MRI particularly dedicated systems for their optimal cost advantage ratio and the easy in mounting them, that can allow to place them at peripherical clinical institutes for the benefit of patients, at present the MRI imaging method is still used in a traditional way, i.e. images are read and analysed from a mere morphologic point of view like radiologic images, therefore above advantages of MRI method are not fully used.

Like conditions are present in the ultrasound field for example as regards heart diseases. As already said above, therefore diagnostic images show a dislaying of the detected datum. The provided representation does not show the only possible displaying form, and often it is not the one direclty correlated with the analysed physiology or disease. Similarly the datum itself is the one directly acquired by the device and it is not a measure closely related to functional characteristics of the organ necessarily or it cannot be directly correlated to the actual physiopathologic situation. These limits can be overcome only by integrating the available echocardiography diagnostics with the additional information value obtained by quantitative evaluating systems able to automatically measure functional cardiac parameters (perfusion, hematic volumes, hematic flows, ejection portions). The quantification is obtained by processing data of ultrasound examinations for providing the cardiologist with an objective numerical support for correctly expressing the diagnosis, for a more careful prognosis definition, and for a precise monitoring of the rehabilitative therapy.

According to prior art, for the above processing there are available reliable quantification systems for various functional parameters working with manual processes and so with slow and rough ones.

There are also available processing institutes with CAD functionalities working in a centralized and remote way with respect to the apparatus for acquiring data such as described above. It is important to consider that such processing means do not consider the datum acquired by the apparatus as the final result, but as a starting point for subsequent processings instead, by means of mathematical techniques and applications of physic laws, aiming at evaluating objective parameters, related to the disease, decreasing to a minimum the dependence between operators of the diagnostic process.

From the above it is clear that at present many so called CAD systems (computer aided diagnosis) are known which systems work on the basis of different methods for processing image data that are limited in processing diagnostic images, that is image data, in order to recognize from images shapes and objects shown in images or predetermined qualities or kinds of shown objects such as for example healthy tissues or tissues suffering from diseases or lesions. In order to obtain required information or at least predictive or classificating indications or possible morphologic quantities or physical parameters from image data, currently known CAD systems use different kinds of known algorithms. However algorithms are relatively complex to be applied and images are generally analysed off-line by a specialized institute that just receives image data files and processes them then giving desired output data.

Apart from the lack of a direct collaboration between the radiologist i.e. between personnel assigned to acquire images, the physician evaluating images and defining the diagnosis and personnel specialized in processing images for obtaining additional information from the CAD system, there is no direct interaction between the apparatus acquiring images and the CAD processing system. Moreover the centralized off-line system often does not have at disposal a complete database of data about the individual patient which data can be of primary importance both for acquiring subsequent diagnostic images and for processing them since they certainly help in informing or adjusting processing systems and i.e. algorithms aimingly requiring a learning or configuration about details of the patient history. A further drawback is the fact that results from the analysis of the off-line CAD system cannot be used immediately or within a time length consistent with the length of the examination therefore the patient must return or repeat the examination consequently having an increase in examination costs. Finally but not of minor importance there is the fact that a centralized processing system with CAD functionalities is often redundant and it is not specialized in aimingly analysing or processing diagnostic images for the specific disease therefore for specific information searched in images.

The aim of the present invention is to provide a system for determining diagnostic indications of the type described hereinbefore and that by means of relatively simple arrangements allows to provide the radiologist and the clinician with a diagnosis supporting and quantification instrument , as possible, for the possible pathologic state.

Moreover a further aim to be simultaneously achieved is the fact of providing the possibility of a direct synergic interaction between means treating and processing image data for determining indications helping the diagnosis and means acquiring image data such to obtain a synergic optimization of all parameters or acquiring settings in order to achieve the best final result.

The invention achieves the above aims by a system for determining diagnostic indications which system comprises:
At least an apparatus for acquiring diagnostic images; and
Image processing means for recognizing and measuring qualitative, quantitative, morphologic and/or dynamic characteristics of one or more objects reproduced in acquired images by pixels, or voxels or image data thereof;
processing means and the apparatus for acquiring diagnostic images being integrated within the same device and the processing of the image or images being carried out directly at the end of the acquisition of the image or images as the final and/or intermediate step of the diagnostic imaging session or process.

Particularly with reference to elements of the apparatus for acquiring images by nuclear magnetic resonance and of processing means, the invention provides means acquiring magnetic resonance signals and determining image data therefrom , means storing said image data, means generating MRI images from image data and displaying them and means processing digital images comprising at least means determining functional morphologic characteristics of objects reproduced in images from image data and/or means determining qualitative and/or quantitative characteristics of the type of objects reproduced in images from image data and classificating them on the basis of said qualtitative and/or quantitative type characteristics, to be integrated in the same device and wherein the system provides as output one or more acquired images or a sequence of acquired images and functional morphologic information and/or information about qualitative and/or quantitative characteristics of the type of objects reproduced in images and about the classification thereof on the basis of said qualitative and/or quantitative type characteristics.

Therefore the invention allows to provide a single diagnostic apparatus carrying out both tasks merely acquiring, generating, storing and displaying images such as for example the acquisition of nuclear magnetic resonance images and/or the acquisition of ultrasound or echocardiographic and/or radiographic images and tasks processing image data with CAD functionalities, i.e. processing functionalities that from image data allow to determine indications about the probability of the presence or absence of a disease and/or about the evolution degree of the disease.

Typically the system provides image data to be directly used in order to obtain said diagnostic indications by their direct processing.

According to an alternative embodiment, the system provides image data to be subjected to different processing processes each of which is intended to prepare the image or images and/or image data for determining and/or extracting therefrom numerical values of parameters measuring pathologic conditions (absence/presence and/or evolution degree of the disease) or each of which is intended to determine numerical values of parameters measuring pathologic conditions by images or by image data.

In this case it is possible to provide different chains for determining or extracting different parameters measuring pathologic conditions from image data and/or from images, which chains work in parallel one with the other and each one provides values for one or more numerical parameters measuring the disease in a independent way one with the other.

These processing processes are carried out by one or more sub-sections and in turn they can be composed of various process steps treating images and/or image data and moreover they can be numerical input data of further processing means working on the basis of classification algorithms and/or predictive algorithms, which provide final indications about the absence/presence of diseases and/or about their evolution degree.

Numerical parameters measuring pathologic conditions are different depending on the type of disease that is searched or examined and obviously they have to be supported by a clinical validation with respect to the real ability in indicating the presence/absence of the disease or the evolution degree of it.

There are many processes treating images and/or image data for obtaining information that can be numerically coded from images and are known and widely used for treating the image in various fields and not only in medical ones.

So for example depending on the type of parameters to be determined from images it is possible to use many of these image processing processes even in a linked way.

A typical mode in processing images is for example the extraction of dimension measures, such as length, width, diameter, surface, volume and other dimension measures of objects reppresented in images. However in order to reconstruct these data from images it is initially necessary to provide the recognition of image areas or image volumes, depending on the fact if they are two or three-dimensional images, and so of subsets of pixels, voxels or image data representing objects in images.

This is obtained by means of a segmentation process. Particularly the segmentation allows to identify subsets of pixels, voxels or image data, which subset elements have such characteristics to be considered as being part of an individual object. The following step obviously requires the univocal identification of the subset of pixels, voxels or image data with a real object that is present in the area or volume of the body under examination of which the image or images has/have been acquired.

As it will be seen below in more details there can be provided various verification processes in order to guarantee that the process segmenting and identifying objects reproduced by subsets provided by the segmentation are correct.

The image or images and/or image data can be subsequently further subjected to the segmentation and processing by rendering tools. This technique, that is known, allows virtual scenarios simulating the reality on the basis of image data and in turn it improves the result of the segmentation, by giving a more userfriendly displaying of images in addition to an improved ability in determining dimension and morphologic characteristics of objects reproduced in the image or images.

Images or image data can be subjected also to further Image processing treatments for recognizing images, such as for example processes known by the names of morphing (modelling), smoothing, pattern recognition, edge detection, feature tracking, registration and any further known image processing technique having some importance.

Above processes for processing images have no value for the diagnostic indication but they provide only to transform images such that can be subsequently processed by automatic or semi-automatic instruments measuring or reading numerical parameters indicating the pathologic condition.

In addition to instruments intended for determining dimensions above instruments can also provide a measure in the form of one or more numerical parameters of the shape of an object reproduced in images and/or of changes to said shape in time both merely regarding the morphologic characteristics and also regarding dynamic characteristics such as elongation and/or compressive stresses and rates at which deformations occur. These last parameters for example are important as numerical parameters for evaluating morpho-functional conditions of the heart activity.

In this case it is possible to use various processing techniques such as for example morphing and/or the feature tracking technique and the following definition of movement and/or deformation vectors and/or vectors of the deformation and movement rate.

Other means for processing images provide to carry out measurements of the time change of the intensity of acquisition signals and/or of the intensity of image pixels or voxels in one or more regions and to determine changing curves from which specific parameters of functions describing said curves and/or other numerical parameters can be determined. An example are the known perfusion curves of contrast media.

Still another example of image data processing intended to provide numerical parameters measuring a pathologic condition is the comparison of contrast maps obtained by image acquisitions carried out at different time moments and/or obtained by image acquisitions carried out by different acquisition techniques or by different settings.

For all above cases determined numerical parameters can be input data of processing means working on the basis of classification and/or predective algorithms, or numerical data of measurement parameters provided by various image processing processes are used for determining in an independent way for each processing process an indication about the presence/absence and/or the evolution degree of the searched disease.

In this case a mode consists in generating a database of known clinical cases for which clinical cases the condition of the presence/absence and/or the evolution degree of the disease is known and for which clinical cases numerical measurement parameters have been determined which can be defined by various processing processes. Therefore a comparison between values of measurement parameters obtained for the case under examination and values of the database of known clinical cases is carried out.

As regards the comparison, it is possible to generate a reference value for each measurement numerical parameter corresponding to a certain function of all values of corresponding parameters of known clinical cases having the same disease and/or the same evolution degree of the disease. In this case it is possible to determine a scale measuring the evolution degree of the disease in addition to a specific reference value for discriminating the presence/absence of the disease.

The comparison between values of numerical parameters measuring the pathologic condition determined for the case under examination and values of corresponding parameters of clinical cases of the database of known clnical cases can be caried out also by using more elaborate comparison tools such as linear regressions, classification networks and/or clustering tools and any other currently known process.

Results regarding the presence/absence of the disease and/or the evolution degree of it obtained for each different image processing process described above are in turn input data of processing means working on the basis of classification and/or predictive algorithms and providing as output the diagnostic indication about the presence/absence and/or the evolution degree of the disease.

In this case input data provided to such processing means can further comprise general information, such as for example personal data and/or other information about life conditions and uses and/or pathologic information of the patient under examination or about his physiologic parameters.

Further data can be considered both in the comparison for determining indications about the absence/presence and/or the evolution degreee of the desease individually provided by each processing process and in determining indications about the absence/presence and/or the evolution degree of the disease provided classification and/or predictive processing means and such data in one case are parameters measuring pathologic conditions computed in previous examinations and in the other case they are indications about the presence/absence and/or the evolution degree of the disease provided by classification and/or predictive processing means in previous examinations and/or as an alternative or in combination they are data relevant to indications of the presence/absence and/or the evolution degree of the disease provided by individual processing processes. This allows also to determine the evolution of the disease in time for the same patient and therefore the need of a therapeutic treatment and the type thereof and/or the efficacy of the therapeutic treatment.

In the following there is a list of documents describing algortihms and processes that are most used as regards various processes that can be used at present for treating images and various classification and/or predictive methods.

A more detailled description of digital image processing on image segmentation can be found in:
1. Middleton I, Damper RI. Segmentation of magnetic resonance images using a combination of neural networks and active contour models, Med. Eng Phys 2004; 26:71-86.
2. Grau V, Mewes AU, Alcaniz M, Kikinis R, Warfield SK. Improved watershed transform for medical image segmentation using prior information. IEEE Trans Med Imaging 2004; 23:447-458.
3. Lucier BJ, Kallergi M, Qian W, DeVore RA, Clark RA, Saff EB, Clarke LP. Wavelet compression and segmentation of digital mammograms. J Digit Imaging 1994; 7: 27-38.

The image registration is described in more details in:
1. Sorzano, CO, Thevenaz P, Unser M. Elastic registration of biological images using vector-spline regularization. IEEE Trans Med Imaging 2005; 52:652-663.
2. Crum WR, Hartkens T, Hill DL. Non-rigid image registration: theory and practice. Br. J Radiol 2004; 77 Spec. No 2:S140-53
3. Park H, Bland PH, Brock KK, Meyer CR. Adaptive registration using local information measures. Med Image Anal 2004; 8:465-473.
4. Kim J, Fessler JA. Intensity-based image registration using robust correlation coefficients. IEEE Trans Med Imaging 2004;23:1430-1444
5. Pluim JP, Fitzpatrick JM. Image registration. IEEE Trans Med Imaging 2003; 22:1341-1343.

Curves picking up contrast media and methods for determining said curves are described in:
1. Daldrup-Link HE, Brasch RC. Macromolecular contrast agents for MR mammography: current status. Eur Radiol 2003; 13:354-365.
2. Sardanelli F, Iozzelli A, Fausto A. Contrast agents and temporal resolution in breast MR imaging. J Exp Clin Cancer Res 2002; 21:69-75
3. Baum F, Fischer U, Vosshenrich R, Grabbe E. Classification of hypervascularized lesions in CE MR imaging of the breast. Eur Radiol 2002; 12:1087-1092
4. Turetschek K, Roberts TP, Floyd E, Preda A, Novikov V, Shames DM, Carter WO, Brasch RC. Tumor microvascular characterization using ultrasmall superparamagnetic iron oxide particles (USPIO) in an experimental breast cancer model. J Magn Reson Imaging 2001;13:882-8
5. Ercolani P, Valeri G, Amici F Dynamic MRI of the breast. Eur J Radiol 1998;27 Suppl 2:S265-71
6. Tofts PS. Modeling tracer kinetics in dynamic Gd-DTPA MR imaging. J Magn Reson Imaging JID - 9105850 RN - 0 (Contrast Media) RN - 0 (Organometallic Compounds) RN - 0 (Radioactive Tracers) RN - 67-43-6 (Pentetic Acid) RN - 80529-93-7 (Gadolinium DTPA) 1997;7:91-101.
7. Griebel J, Mayr NA, de Vries A, Knopp MV, Gneiting T, Kremser C, Essig M, Hawighorst H, P.H. L, Yuh W. Assessment of tumor microcirculation: a new role of dynamic contrast MR imaging. J. Magn Reson Imaging JID - 9105850 RN - 0 (Antineoplastic Agents) RN - 0 (Contrast Media) RN - 0 (Radioactive Tracers) RN - 7440-54-2 (Gadolinium) 1997;7:111-9.
8. Hoffman U, Brix G, Knopp MV, Hess T, lorenz WJ. Pharmacokinetic mapping of the breast: a new method for dynamic MR mammography. Magn Reson Med 1995;33:506-14.

The use of classification algorithms and particularly the use of artificial neural networks, as well as systems coding pixels or voxels for processing an image by means of a classification algorithm are described in:
1. Szabo BK, Wiberg MK, Bone B, Aspelin P. Application of atificial neural networks to the analysis of dynamic MR imaging features of the breast. Eur Radiol 2004;14:1217-1225.
2. Szabo BK, Aspelin P, Wiberg MK. Neural network approach to the segmentation and classification of dynamic magnetic resonance images of the breast: comparison with empiric and quantitative kinetic parameters. Acad Radiol 2004;11:1344-1354.
3. Vomweg TW, Buscema M, Kauczor HU, Teifke A, Intraligi M, Terzi S, Heussel CP, Acehnbach T, Rieker O, Mayer D, Thelen M. Improved artificial neural networks in prediction of malignancy of lesions in contrast-enhanced MR-mammography. Med Phys 2003;30:2350-2359
4. Perez de Alr, Ruiz-Cabello J, Cortijo M, Rodriguez I, Echave I, Regadera J, Arrazola J, Aviles P, Barreiro P, Gargallo D, Grana M. Computer-assisted enchanced volumetric segmentation magnetic resonance imaging data using a mixture of artificial neural networks. Magn Reson Imaging 2003;21:901-912.
5. Lucht RE, Knopp MV, Brix G Classification of signal-time curves from dynamic MR mammography by neural networks. Magn. Reson Imaging 2001;19:51-7
6. Markopoulos C, Kouskos E, Koufopoulos K, Kyriakou V, Gogas J. Use of artificial neural networks (computer analysis) in the diagnosis of microcalcifications on mammography. Eur J Radiol 2001;39:60-5
7. Vergnaghi D, Monti A, Setti E, Musumeci R. A use of a neural network to evaluate contrast enhancement curves in breast magnetic resonance images. J Digit Imaging 2001;14:58-59
8. Adbolmaleki P, Buadu LD, Maderimansch H, Feature extraction and classification of breast cancer on dynamic magnetic resonance imaging using artificial neural network. Cancer Lett 2001;171:183-91
9. Chen DR, Chang RF, Huang YL, Chou YH, Tiu CM, Tsai PP. Texture analysis of breast tumors on sonograms. Semin Ultrasound CT MR 2000; 21:308-316
and more generally in:
1. Buscema M. A brief overview and introduction to artificial neural networks. Subst Use Misuse 2002; 37:1093-1148
2. Haykin S. Neural Networks:A Comprehensive Foundation, 2 ed. New York: Macmillan 1999
3. Buscema M. Artificial Neural networks and complex social systems. I. Theory. Subst Use Misuse JID - 9602153 1998;33:v-xvii, 1-220 FAU - Bu
4. Buscema M. Theory: Foundation of Artificial Neural Networks. Substance Use & Misuse 1998;33:28-98.

As regards other processes for processing digital images known as morphing and rendering ones it is possible to generally refer to documents:
1. Digital Image Processing Jdhne Bernd Springer 2005.
2. 3-D Image Processing Algorithms, Nikoladis, Nikos; Pitas Ioannis, Wiley-Interscience 2000.
3. Non linear Model-Based Image/Video processing and Analysis, Pitas, Ioannis; 2001 Wiley-Interscience.

As regards specific CAD analysis means for images constisting in Bayesian classifiers and so called Support vector machines, in this case they are linear classification machines known for example from Nello Cristianini and John Shawe-Taylor. An introduction to Support Vector Machines and other kernel-based learning methods, Cambridge University press 2000 ISBN 0-521-78019-5 Chih-Wei Hsu, Chih-Chung Chang and Chih-Jen Lin, Practical Guide to Support Vector Classification Department of Computer Science and Information Engineering, National Taiwan Univeristy 2001 Taipei 106, Taiwan that can be downloaded from www.csie.ntu-edu.tw/cjlin/libsvm.

As regards the device, the integration is quite simple, since processing means and various processes that provide CAD functionalities are composed of so called software "tools", i.e. programs treating data that can be loaded in memories of a computer and executed by it, for example a computer of the personal computer type or the like. Said programs work functions that treat data according to different type of algorithms which determine an operation on image data according to one or more of the above type, such as segmentation, modelling, registering, etc. It is to be noted that a part of processes for generating and displaying images that are typically carried out by the apparatus for acquiring MRI images can be composed of software i.e. computer programs that are executed by computers and that tomographs for acquiring MRI images typically comprise computers in the form of personal computers or computers with dedicated hardware able to execute another code. Therefore the integration of functionalities of CAD systems in the tomograph from the constructive point of view requires at most an upgrade of mass and buffer memories and/or of the computation skill, i.e. of the processor and of elements cooperating with it, as well as the development of CAD processing software.

By integrating means acquiring, generating and displaying MRI images with image processing means having CAD functionalities or of the type defined above, the invention allows to easily expand the kind of data to be processed.

According to an advantageous embodiment data acquired by the tomograph or by the image acquiring apparatus and subjected to the analysis by processing means having CAD functionalities are not only images, i.e. image data, but also intermediate data that can be obtained during MRI scans, such as for example the acquisition signal both considered in its totality and at intermediate processing stage of the step extracting the image datum.

The integration of image acquisition means with analysing means having CAD functionalities according to the present invention leads still to a furhter considerable advantage. This advantage is the fact that it is possible to have a direct feedback interaction communication between said means for acquiring images and said processing means with CAD functionalities, which interaction allows to automatically and/or semi-automatically optimizing settings of acquiring means upon the control of processing means and possibly also viceversa.

Particularly said interaction between means for acquiring, generating and displaying images with processing means having CAD functionalities and/or with individual processes determining values of numerical parameters measuring pathologic conditions, provides processing means having CAD functionalities and/or individual processes determining values of numerical parameters measuring pathologic conditions to comprise also means for analysing images or image data and/or acquisition signals with regard to specific quality parameters and means for automatically or semi-automatically determining new acquisition settings such to improve at some probability images and/or image data and/or acquisition signals as regards the needs of processing means and not of the human operator i.e. of the image displaying.

The practical realization can provide means for generating acquisition settings that are controlled by means for verifying the quality that are associated and/or present in processing means.

In the particular case of MRI image acquisition, it is well known that the excitation of resonance signals can occur according to different protocols that are characterized by different parameter settings and different acquisition sequences. Parameter settings and the choice of one acquisition sequence or of the other one determines not only the quality of the image, as regards the resolution and/or contrasts and/or signal/noise ratio or the presence of artefacts, but also information that can be seen or extracted from acquired images and the length of the acquiring process.

According to the above characteristic of the interaction between acquiring means and means purely dedicated to the processing with CAD functionalities the optimization of all parameters aiming at the best final result is "guided" by processing means with CAD functionalities. That leads to the advantage of a better functionality acquiring, generating and displaying images and a more reliable CAD functionality, since means having CAD functionalities carry out the adjustment on the basis of specific requirements of said means with CAD functionalities for optimizing their task.

Obviously such interaction is also possible for example by providing processing means with CAD functionalites in combination with a device for acquiring images by ultrasounds, such as a traditional echograph or a radiologic apparatus. In this case the interaction will concern the control of the ecograph or the radiologic apparatus by processing means in order to modify for their optimization the acquisition parameters of images that can be changed as regards the specific type of image acquisition technique.

Therefore in particular the invention provides a system of the type described hereinbefore wherein image processing means for recognizing and measuring qualitative, quantitative, morphologic and/or dynamic characteristics of one or more objects reproduced in acquired images and/or of image data and/or of acquisition signals from which image data and said images are extracted have means for analysing one or more qualitative parameters of images and/or of image data and/or acquisition signals, which means generate a command changing settings of acquiring means.

When said acquiring means are of the MRI type then the change can also concern acquisition sequences.

In addition to or instead of above criteria determining the command signal changing above parameters and/or image acquisition, image processing means can have means for analysing parameters measuring the reliability of processing results carried out by processing means with CAD functionalities. These parameters generally are statistic parameters automatically determined by processing means with CAD functionalities such as for example fitness values, statistic errors or other parameters.

As regards the change of image acquisition settings, the invention can provide also mathematical means such as for example fuzzy algorithms or genetic algorithms for determining new settings acquiring signals and/or image data such as for example means based on the processing by means of genetic algorithms or other similar algorithms, which algorithms can also be guided by statistic algorithms selecting or predicting new values of acquiring parameters or of acquisition sequences that have a greater probability in providing better image data from the point of view of processing means.

It is possible for the interaction between acquiring means and image processing means to be carried out according to iterative steps, i.e. by providing to carry out different subsequent steps acquiring images and subsequently processing them by processing means with CAD functionalities, as well as qualitatively analysing and determining new acquisition parameters and/or new acquisition sequences and subsequently repeating previous acquisition and processing steps. Said steps can be iteratively repeated up to a certain number of times till processing means reach predetermined minimum threshold values of statistic reliability parameters of results.

From the above the synergic advantages deriving from the fact that the CAD system is integrated in the MRI system are clear. By that the two systems can work in an optimized mode.

According to a further advantageous characteristic, the system provides means for storing a database of diagnostic images and of processing data with CAD means relevant to each patient.

Storing means can be composed of means integrated or resident in the system computer or also of movable and portable storage media in combination with readers of said media provided in the system.

In addition to diagnostic data i.e. to images and results of image processings, the patient database can comprise additional data, such as image acquisition settings, used processing means, conditions of the patient at each image acquisition defined on the basis of other physical or physiological parameters.

By means of the patient database, it is possible to repeat specific image acquisitions and corresponding processings with processing means having CAD functionalities in different time moments even at relatively long time intervals, such as days, months or years and to determine the evolution of a condition by the comparison of image data and/or of processing results. In the technical language this mode is known as follow up and it allows to verify the development of a disease for example in order to determine the kind of operation to be carried out and/or to verify the efficacy of a therapy in use, for example by changing it in the case of a slow or inexistent development as regards recovery, such as for example changing the dosage of drugs and/or the kind of active principle. Similarly it is also possible to control the evolution of side effects of the therapy and to possibly act in limiting it or in reducing the dosage if side effects are worrying.

To this aim, the diagnostic imaging carried out on anatomical regions subjected to searched diseases can be integrated with physiological data and/or images from other anatomical regions which can be acquired in a different form or by different acquisition methods and which anatomical regions are those in which or for which potential side effects are provided.

These data can also be stored in the patient database. That is possible by using data coding protocols known as DICOM and constituting a standard in the medical field.

When comparing MRI diagnostic images and results of their processing with CAD processing means first the system verifies acquisition settings and processing means used in the previous diagnostic image acquisition or acquisitions and processing or processings and it uses the same settings even for the new image acquisition and for processing them. Image data and/or processing results therefore are compared one with the other and a compatibility verification is carried out intended to indicate if the possible change of conditions determined by the subsequent examination, i.e. by the subsequent image processing and acquisition session, is consistent also with a drastic aggravation or with a drastic and sudden recovery and it indicates conditions of image data and results of their processing that can be consistent with or not consistent with image data and results of their processing relevant to a previous examination, advising or automatically carrying out a process repeating the acquisition of the image or images and the processing thereof in case with modified sequences and/or parameters and/or with processing means that are modified and/or different ones and/or integrated with one or more different processing means in addition to the already used ones.

Moreover a further characteristic of the invention is the fact of providing the integration of image data or other data obtained by the magnetic resonance technique with image data or other data obtained by different techniques for acquiring diagnostic images, such as for example radiologic or ecographic techniques, or the like and that both as regards image data and/or other parameters directly deriving from acquired signals and/or as regards processing results of said image data and/or acquired signals.

Both for correctly carrying out the comparison in the follow up process and for the integration and/or merging of information obtained by different acquiring techniques it is important to provide means automatically or semiautomatically registering images that are generally also known.

Still according to an advantageous characteristic of the invention, processing means are composed of one or more computer programs that can be executed by a computer such as a PC or the like.

Particularly the architecture of said means comprises a managing logic or program and a set of one or more programs each of which is intended to carry out a different process for processing images or image data or a specific control step such as the qualitative verification and the change of acquisition settings, as well as the repetition of the acqusition.

In this case each processing process is a software application that can be executed upon the call-up by the logic program. As it is clear from the above, each application software is composed of a program intended to execute image and/or image data and/or acquisition signals processing according to specific algorithms and so it is the same for any specific diagnostic use. However the specific disease and/or the specific mode for acquiring images requires at least partially the application to be specialized for steps necessary for the examination of pathologic conditions of the specific disease and for the acquisition according to the specific image acquisition method.

In this case, the invention provides to generate a set of application softwares, each of which executes a specific processing process and as regards characteristics in common to all kinds of diseases it is managed by an intermediate managing program, i.e. a so called diagnostic middleware, whereas as regards options specific for the disease under examination, the application program i.e. corresponding routines are directly managed by the operating system.

Therefore according to the invention the set of functionalities transversal to different application fields are joined together in a single software product, a middleware for medical imaging, intended to support development steps in different application environments. Such a type of middleware is a program able to place a series of function blocks for the common use at disposal for various application fields. Common functionalities used by applications for the diagnosis are taken by the medical middleware. Only remaining contingent functionalities, the basic ones or functionalities that are highly specific ones, are directly required by the basic operating system by means of classic approaches.

Thus advantages typical of software engineering techniques can be introduced when designing the basic software for medical equipment. Particularly:
- re-use of the code with a consequent increasing of the efficacy of development steps;
- centralized process for releasing the code (i.e. a change on one component of the middleware will affect all applications using such functionality);
- increasing of the abstraction level during development steps (i.e. the programmer can think in terms of high level abstractions, and that is by entitites of its application domain, thus taking no interest in lower level details).

Within the development and realization the middleware has the following advantages:
- razionalization of implementative efforts in making medical apparata;
- ability of the information infrastructure to accelerate the production of new biomedical imaging apparata;
- the possibility in the future to make the production of innovative applications easier by reducing efforts in re-implementing already existing functionalities, and by limiting the development to a step integrating pre-existent components.

Further characteristics of the invention are object of the subclaims.

Characterstics of the present invention and advantages deriving therefrom will be more clear from the following description of some embodiments with reference to annexed drawings wherein:
Figure 1 is a block diagram of the principle structure of the system according to the present invention, with particular reference to an apparatus for acquiring MRI images.
Figure 2 is an example for realizing the system according to the present invention wherein means acquiring MRI images and means processing images with reference to CAD functionalities are composed of a software and hardware combination and particularly of application programs that are executed by a computer in common both to acquiring means and to processing means.
Figure 3 is a block diagram showing the principle of processing means for recognizing objects from the morphologic and/or dynamic-functional point of view.
Figure 4 is a block diagram showing an example of the segmentation process, wherein the traditional process segmenting the image is integrated with morphologic and/or dynamic functional data typical for real objects reproduced in images.
Figure 5 is a block diagram of means processing images that are specifically provided in the system according to the present invention for the diagnostic help by a computer.
Figure 6 is a block diagram of the system according to the present invention regarding an example of automatic or semiautomatic interaction means between means acquiring images and means processing images for optimizely setting particularly acquiring means with reference to processing means.
Figure 7 is a block diagram of the structure of processing means in the system according to the present invention.

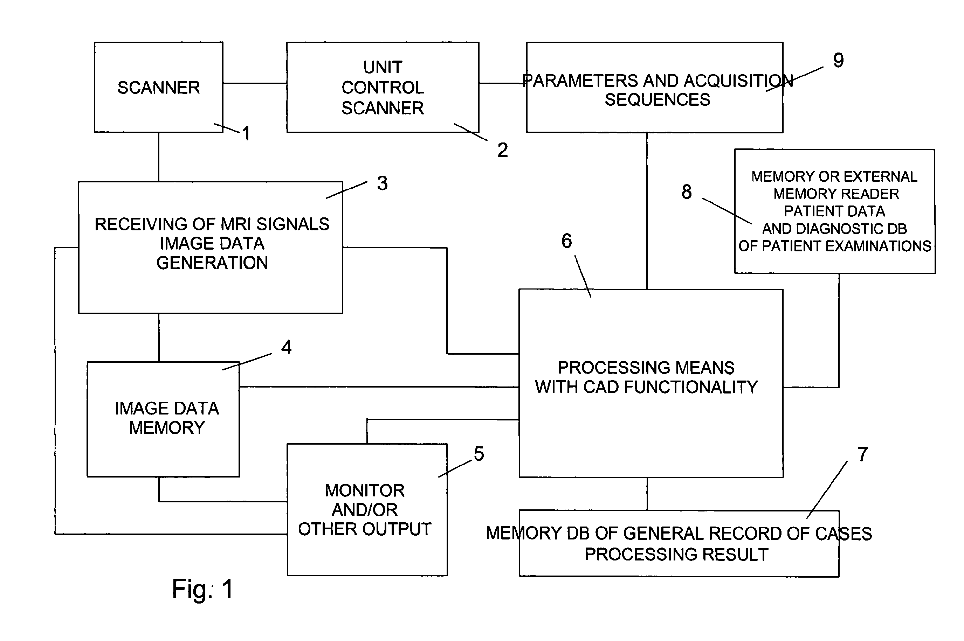

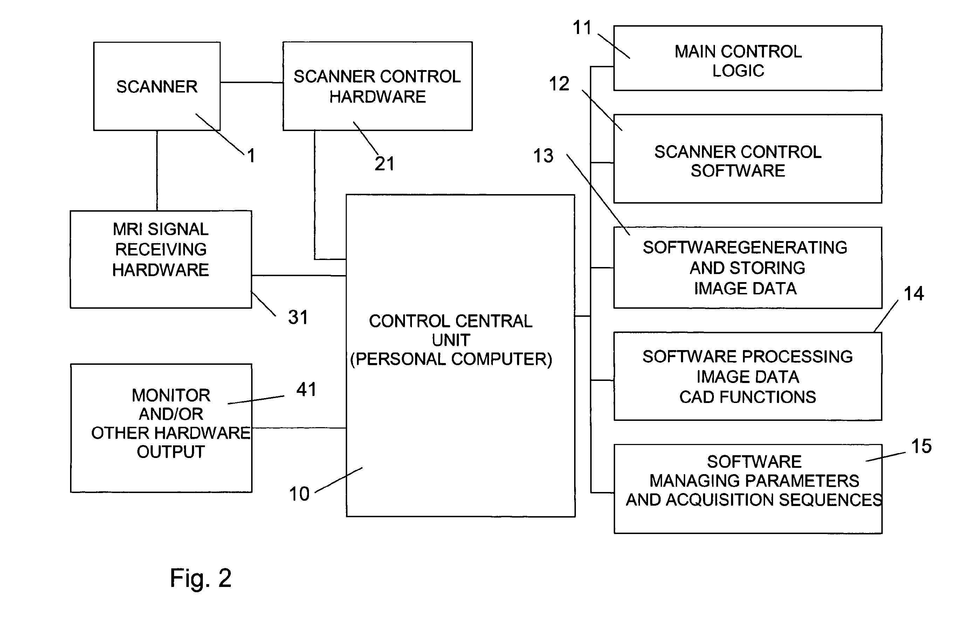

The example of figures 1 and 2 specifically refers to a system comprising a unit for acquiring images by nuclear magnetic resonance. This is not to be considered as limitative as it is possible to easily replace the MRI apparatus with an echograph or with a radiologic device.

With reference to figures 1 and 2 a diagnostic helping system comprises an apparatus for acquiring images, particularly an MRI apparatus, i.e. intended for acquiring images by nuclear magnetic resonance, wherein a unit for processing acquired images is integrated providing to extract from images information about the state or conditions of objects reproduced in said images helping the diagnosis.

For example the system according to the present invention can be intended for recognizing the presence of rheumatic diseases and in the specific case of hand and knee regions or of orthopedic diseases in the same regions or in other anatomical regions such as the backbone. However above principles that will be described in more details are applied to any anatomical regions and to any disease that somehow or other can be detected by used image acquiring means.

At present different means for processing images are known per se having different functionalities and intended to define or to extract from image data specific characteristics of images and/or of objects reproduced in the image. These means are composed of algorithms processing images i.e. image data. In order to better understand the following description it is to be noted that digital or digitized images are composed of individual points called pixels in two-dimensional images and voxels in three-dimensional images. The visual information of the image is determined by the appearance of each individual pixel or voxel with respect to surrounding pixels or voxels. The appearance of each individual pixel or voxel can be defined by specific parameters that are univocally defined in colorimetric systems by using different kinds of definition spaces like the well-known ones defined for example as HSV or other ones.

Therefore the image is defined by a set of pixels or voxels ordered in the form of a two-dimensional array as regards pixels and a three-dimensional array as regards voxels. Therefore to each unit image element i.e. to each pixel or voxel there is associated a set of parameters describing their appearance. The set of these parameters that are grouped together in the form of a two-dimensional or three-dimensional array depending if they refer to pixels or voxels, describes and defines in the numerical form the two-dimensional or three-dimensional image and specific values of parameters correlated to each pixel or to each voxel are generated from signals received from the object under examination, i.e. a body under examination or a part of the body under examination by extraction functions that are defined by the kind of used signal and/or by the acquisition mode.

Therefore algorithms processing images work on said sets of image data in the form of two-dimensional or three-dimensional arrays and such operations are substantially equivalent or can be defined as operations carried out on the image since as mentioned above image data are only a numerical representation of the image.

Therefore processing means with CAD functionalities can be advantageously composed of computer programs that execute operations defined by the algorithms processing images and which programs are executed by computers and particularly by Personal Computers or computers with similar system architectures.

The unit for acquiring MRI images shown in the example of Figure 1 comprises a scanner 1 with a magnetic structure and with various gradient, transmitting and receiving coils, as well as with units generating image data from received resonance signals. Therefore the scanner 1 represents the magnetic structure, gradient coils, the receiving coil and the transmitting one and further possible devices or means for acquiring resonance signals. 2 and 3 indicate means controlling the scanner and means receiving signals and generating image data and both are generally composed of electronic devices. These means 2 and 3 can be also composed of combinations of hardware and software means and as it will be seen below of software means loaded in a general hardware executing the software, such as a computer or also a personal computer.

Image data generated by the unit 3 are stored in a memory 4 and therefore they can be called up for being displayed at any moments by means of the monitor 5 or other output means or they can be immediately displayed apart from the storage.

The unit processing image data having CAD functionalities comprises processing means with CAD functionalities that are indicated by 6 and to which there are provided or which call up image data of one or more images to be subjected to processing processes from memory 4, or from the unit receiving and generating image data 3 and/or possibly even from the monitor or from further possible output means 5 when they allow it. As it will be seen below processing means can provide different kinds of outputs that as regards the form and contents depend on specific functional requirements. Processing results can be in the form of alphanumeric data and/or as an alternative or in combination in the form of static images and/or as an alternative or in combination in the form of dynamic images i.e. image sequences like films or the like. Results in the alphanumeric form and/or in the form of image or images can be displayed on the monitor 5 or printed or personnel can access to them by other output means. Moreover in combination or as an alternative results are stored in a memory 7 of general record of clinical cases that as it will be seen below has both the task of having data of various examinations for each patient available in order to allow the identification of the time evolution of patient conditions and the task of increasing a database of known record of cases that is necessary for training expert algorithms used by processing means with CAD functionalities 6 and improving their performances in time and by the use by means of a constant learning. As an alternative or in combination it is possible to provide also a reading/writing unit 8 of an external portable memory, such as a tape, a floppy-disk, a writeble or re-writable CD or DVD or a so called smart card wherein data of each examination for each patient are stored together with other data obtained from other examinations.

Thanks to that it is possible to guarantee patient privacy, limiting data relevant to patients introduced in the database 7 of record of cases and keeping possible more sensitive and private information both personal and diagnostic ones only inside the portable memory in patient's hands, therefore the access to these data is legally possible only with the express will of the patient and only when the examination is carrying out. This mode can be carried out by means of the integration of image processing means with acquisition means and by the substantially immediate execution i.e. within the same examination session of said processing. Therefore, as it will be seen below, when means processing images provide the use of predictive or classification algorithms for image data for indicating and/or classificating predetermined searched characteristics of objects represented in images and particularly an indication of a probable presence or absence of a rheumatic disease and/or of the degree of such pathologic condition and/or the probability of developments with the disease improving or worsening, the use of data deriving from further different analyses or sources in combination with specific acquired images can be very important for the accuracy and reliability of the prediction and/or classification.

The practical realization of the system according to the present invention requires the combination of hardware units with software units and such combination is particularly advantageous in the specific medical field relevant to diagnostic images.

As already said above, apparati for acquiring images in nuclear magnetic resonance are composed of combinations of operating units that are controlled by electronic control units, at least a part of said electronic control units being composed of one or more computer programs that are executed by a computer, that is made in particular with an architecture of the type similar to the one of personal computers or it is a real personal computer. In this case means for processing images having CAD functionalities can be composed of programs for processing images, i.e. image data that can be executed by a computer and particularly by a computer working in parallel to the one dedicated to the control of operating units for acquiring images or by the same computer.

Individual programs can be provided in the form of separated tools processing images or image data that can be used individually or as linked together according to different orders and in any combination or sub-combinations of different tools correspondingly to results desired from the processing.

Figure 2 schematically shows an example of an apparatus incorporating the system of the present invention.

The apparatus provides a central control unit 10 composed for example of a personal computer or of two or more personal computers working in parallel one with the other.

In suitable memory areas or in dedicated memories there are stored different programs each of which is intended for executing specific tasks and is executed by the central unit 10 when such task is expressly required by a command of the personnel or when it is part of an operating sequence that is automatically executed and controlled by a control logic represented by the memory area 11.

The memory area 12 represents the program controlling the MRI scanner, such term scanner has to be intended in its wider meaning as already said above and so it comprises operating units mainly being the magnetic structure, gradient coils and the transmitting coil. Further operating units optional or having secondary importance are associated thereto. Moreover the scanner comprises also operating units necessary to detect and receive signals in this case magnetic resonance signals.

Operating units included in the scanner are controlled by hardware control units indicated by 21 and whose operation is controlled by a program controlling the scanner indicated by 12. Output resonance signals from the scanner are processed by a MRI signal receiving unit generating image data from MRI signals and comprising hardware means indicated by 31 and are controlled by a program generating and managing image data such as in particular the storage indicated by 13. Means processing images and/or image data are composed of one or more program modules that are executed by the same central control unit 10.

Image data that are not subjected to processing and/or processing results having CAD functionalities both in the form of alphanumeric indications and in the form of modified or reconstructed images are emitted by means of output devices 41, such as for example one or more monitors and are controlled by the central unit 10 and by special programs controlling the output devices.

With reference particularly to figures 1, 2 and 6 there is provided an interaction made by means of a feedback line between means acquiring images, particularly MRI ones, i.e. the portion of the apparatus for detecting MRI images and means processing images and/or corresponding image data with CAD functionalities in order to determine indications helping the diagnosis from images and/or corresponding image data. It allows to obtain an adaptation and/or a direct optimization between means acquiring images and means processing them, such to set at best means acquiring images with reference to optimal requirements of processing means.

Similarly to what described above means for the interaction between means acquiring images and means processing them are also composed of a combination of hardware and software means.

Referring to figure 1 showing a principle diagram of a system according to the present invention, there is provided a unit generating and/or setting parameters and nuclear magnetic resonance image acquisition sequences that is indicated by 9 and receiving setting command or setting changing command signals as input on whose basis it generates parameters setting the scanner 1 and/or it defines image acquisition sequences and it provides said parameters and said sequences to the unit 2 controlling the scanner for acquiring images.

With reference to figure 2 and as already mentioned above said unit 9 is composed of a combination of hardware means and software means. Considering the example of hardware/software architecture of figure 2 for the system according to the present invention in a memory or in a dedicated memory area there is stored a software optimizing parameters and acquisition sequences indicated by the box 15. Such software can be executed by the central control unit 10 managing both the interface between the software processing image data having CAD functionalities 14 with said software optimizing acquisition parameters and acquisition sequences 15 and the generation and sending of setting or changing setting commands of parameters and acquisition sequences to the unit 21 controlling the scanner 1.

It is possible to provide different modes for specifically carrying out the interaction between the image acquiring apparatus and image processing apparatus with CAD functionality above all as regards criteria on the basis of which acquisition parameters are determined or changed.

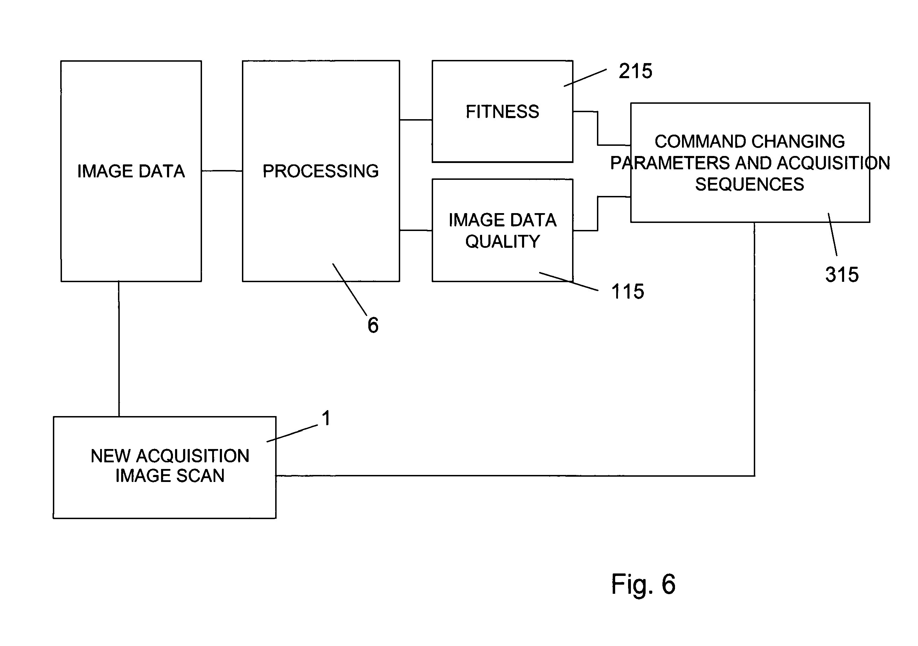

Figure 6 is a block diagram of a sub-system for the interaction between the apparatus acquiring images and means processing them.

Image data from the receiving unit 3 are provided to the processing unit 6. As it will be more clear below, CAD processing means obtain information from image data by using algorithms of the statistic, predictive, or evolutive type that however provide also reliability or error parameters of output data together with output data. Moreover resonance signals from which image data are extracted can be analysed as regards their quality with reference to some quantities important for the image quality and particularly they are the signal/noise ratio, resolution, contrast but are not limited thereto.

Therefore interaction means can have two or more different sections determining parameters and acquisition sequences one of which working on the basis of reliability parameters of the output of CAD processing means and the other one working on the basis of quality parameters of resonance quality and/or image data such as indicated by 115 and 215. Therefore the two sections provide information regarding the quality of image data with reference both to mere acquisition steps and to the reliability of output data of processing means, therefore in section 315 by the comparison with threshold values for relative reliability parameters and/or for quality parameters of resonance signals and/or of image data new values of setting parameters and/or changes of acquisition sequences are established intended to obtain a change in the reliability and/or quality parameters regarding the improvement of said parameters.

Therefore such new setting parameter values and/or such changes of image acquisition sequences are provided to the scanner 1 in the form of a command changing parameters and setting sequences for carrying out a new image acquisition scan.

Image data obtained by the new scan can be subjected again to steps described above for verifying if changes of settings have led to expected improvement results with regards to the quality of resonance signals and/or image data and in combination or as an alternative with regards to reliability of outputs of processing means.

Above steps can be also iteratively repeated till a certain amount of iteraction steps is carried out and/or till the quality of resonance signals and/or image data and/or the reliability of outputs of processing means reach or overcome predetermined minimum threshold values.

As regards changing values of setting parameters and/or the choice or structure of acquisition sequences, such values or such data can be determined also by using statistic evolution systems, such as genetic algorithms or other similar algorithms by means of which new setting parameters and/or new acquisition sequences are determined that are then used for steps acquiring resonance signals and image data.

With reference to setting parameters and/or to acquisition sequences determined on the basis of feedback by processing means as indicated above, and especially if such values or such sequences are determined by evolutive algorithms, so it is possible to introduce a test acquisition step wherein a reduced amount of resonance signals is acquired or image data or sequences are carried out for a limited field of vision with respect to the desired one, in order to reduce acquisition time. Resonance signals and/or image data obtained by said test acquisition steps are therefore subjected to quality control and the obtained quality is compared with the one obtained in the scan of the preceding acquisition step. When setting parameters or sequences used in the test acquisition step provide quality improvement with respect to the ones of preceding acquisitions therefore the complete acquisition step is carried out, whereas when such quality improvement of resonance signals and of image data is not detected the step computing new acquisition parameters or new sequences is again carried out without carrying out the complete image acquisition step and so drastically reducing test time.

The above allows to obtain a direct and immediate interaction between acquisition means and processing means with processes iteratively carrying out the optimization of acquisition means with respect to processing means and to results provided therefrom. It is also possible for the interaction to occur in a bidirectional way. Particularly when processing means require such a setting of acquisition parameters and/or of sequences to determine acquisition times that are very long or greater than specific thresholds, therefore the acquisition apparatus communicates to CAD processing means that required acquisition parameters and/or sequences cannot be executed and it requires a change of processing means as regards carrying out particular processes treating image data and/or resonance signals that usually are not carried out, such as not linear filters or other image treatment processes, by means of which the extension of overall examination time is greater than time provided on average, but smaller than the ones that would be necessary by changing acquisition parameters and/or sequences such as required by processing means in the configuration conventionally provided.

Such condition can be easily carried out, since as it will be more clear below and however such as it already results from figure 2, processing means are composed of a plurality of software tools or modules that can be independently addressed one with respect to the other and that can be interfaced one with the other in any combinations and amount as regards operations to which image data and/or resonance signals are desired to be subjected.

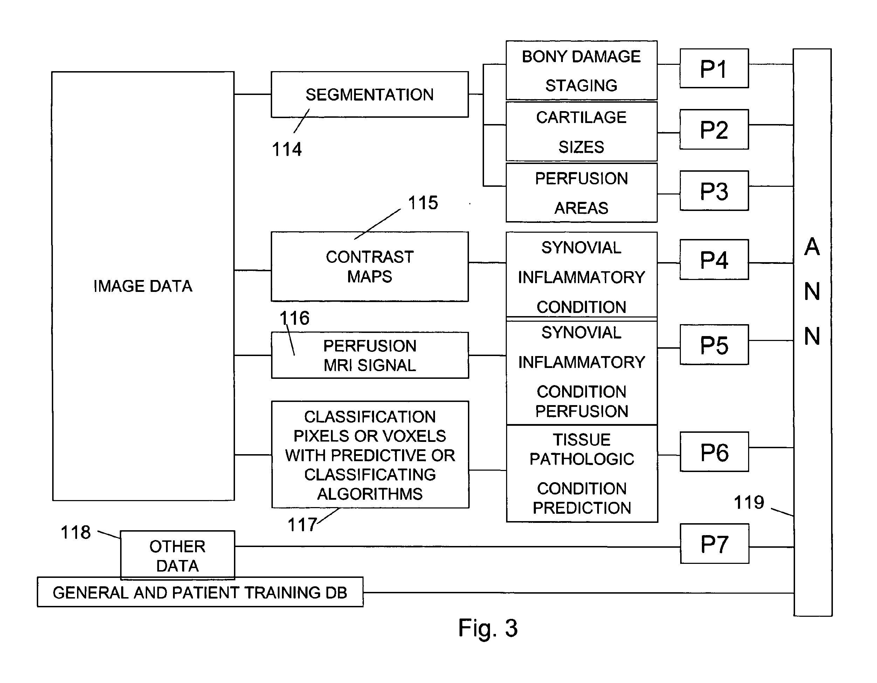

Figures 3 to 5 schematically show an example of different processes treating resonance signals and/or image data that can be provided in combination one with the other such as tools (instruments) of processing means.

In such figures reference will be made to particular methods treating images working on the basis of different algorithms types. These algorithms and the corresponding application to the general image treatment is known per se and below there is provided a list of pubblications wherein different types of algorithms and methods for processing images are described in details, i.e. different image processing tools whose specific structure is not the object of the present invention that supposes its existence. However examples of image processing algorithms expressly mentioned are not to be considered as limitating the general protective sphere.

Therefore, with reference to figure 3, the system according to the present invention provides means processing image data having CAD functionalities composed of different software modules or programs each of which is a processing tool allowing to measure or determine parameters indicating the rheumatic disease and the evolution stage thereof.

By means of a segmentation module image data and/or corresponding pixels or voxels are divided in subsets, each of which is relevant to a specific object having its own identity independent of sets of image data and/or of pixels or voxels and each of which subsets of image data, pixels or voxels is the reproduction in the image of an independent or individual object being part of the body part or area under examination.

The segmentation allows to mainly define three parameters and i.e. the staging of the bony damage due to the rheumatic disease, the dimensions of the cartilage and to delimit circumscribed regions about which perfusion measurements have to be carried out.

Particularly for perfusion measurements described in details in EP 1.516.582, it is possible to use the segmentation of image or images since it allows to find up-take regions and the measure of the signal trend in these regions at subsequent times, in order to create curves of the signal of up-take region with respect to time starting from the injection of the contrast medium. Such curves are made as much precise as possible by the three-dimensional segmentation of all up-take regions and by the measure of the RM signal of such regions. Measures can be analyzed by automatic algorithms having the aim of defining characteristic parameters of such perfusion, such as for example the up-take rate, the possible stationary condition and the wash-out rate, that as it will be described in more details below will be correlated to other clinical parameters in order to define correlations with the pathologic condition and its potential evolution.

Segmentation is an image processing method known per se and it will be briefly summed up in the following description in order to make the comprehension easier with regards to the integration of this processing tool in the system of the present invention and for obtaining diagnostic quantitative information.

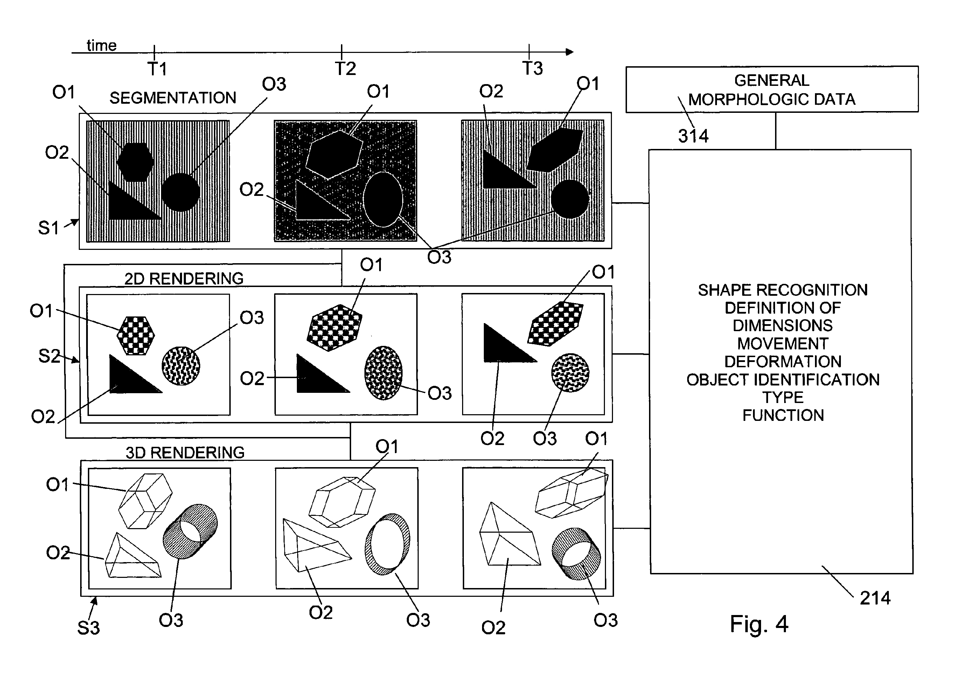

With reference to figure 4, it schematically shows the process for segmenting image data and allowing to carry out subsequent steps recognizing shapes of reproduced objects, dimensions thereof and/or movements and possibly also deformations.

S1 denotes a sequence of images acquired in different subsequent time moments indicated by T1, T2, T3. In this case they are images of the same anatomical region that have been acquired along the same scanning plane for 2D images or within the same scanning volume for 3D images.

As it is schematically indicated there are provided three objects 01, 02, 03 in the region that move and/or are subjected to deformation with the time goes by and representing in images three different objects provided in the volume of the body under examination subjected to image acquisition. These real objects for example can be three different kinds of tissue and/or three different kinds of organs, or the like.

Segmentation is the process for defining subsets of pixels or voxels of images that are in regions or volumes corresponding to the reproduction in the image of the real object. Once said subsets have been defined it is possible to transform each subset in a virtual object therefore having its functional or semantic unit and that is the image of a real object comprised in the plane or volume of which the image has been acquired.

Therefore in each image it is possible to recognize the object and by the comparison with one or more preceding images it is possible to determine the behaviour in time of each object as regards the position, orientation, shape and dimensions.

Once objects and the behaviour in time thereof have been defined as said above, it is possible to provide the generation of a virtual image a kind of virtual copy of the real world wherein objects and behaviours are further highlighted by means of rendering, morphing, smoothing processes and other methods generating virtual realities.

Sequences S2 and S3 schematically show an example of 2D and 3D rendering of the behaviour of objects recognized in the sequence S1 by means of segmentation.

It is to be noted that while in segmented images, contrasts are still defined in the form of real image along the scanning plane or in the volume subjected to examination, i.e. contrasts and the appearance of pixels is directly determined by acquired signals, in images generated by rendering, morphing, smoothing processes or the like, it is possible to change the pixel appearance according to any univocal criteria and particularly in order to make easier the visual reading of the dynamic behaviour of objects and/or their topologic and dynamic interralations for the human operator and that is by defining colorations and intensity changes according to different functions correlating image data and/or by means of smoothing contours and/or by introducing three-dimensional displaying effects such as for example shadings or light blazes and shades of colours.

Rendering and segmentation aim also at making the reading easier, both the automatical and the manual one, i.e. by the operator for example of dimensions of the cartilage and/or stratifications of bony damages and/or dimensions, shape of circumscribed perfusion measuring areas.

By means of segmentation and rendering steps in combination possibly with morphing and/or smoothing treatments, it is possible to univocally give each subset of pixels or voxels to a real object, therefore generating from subsets of pixels or voxels representing the object in said image corresponding virtual objects that are considered as a single set in the following processing and allowing to determine numerical parameters regarding position, orientation, shape, movement in time and shape changes in time of various objects. Moreover by considering that generally reproduced objects are provided with a standard shape from which they are different for secondary variants, it is also possible to provide the comparison of morphologic and dimension data of various objects as determined by images by means of above processes with morphologic and/or dimension data typical of real objects therefore allowing to determine also the kind of object reproduced in the image by each subset of pixels or voxels.

Each object, such as for example each anatomical part has its own individual morphologic and dimension characteristics that by comparing them with shapes and dimensions of objects determined by images allow to verify if the object reproduced in the image coincides with the comparison one therefore also the identity or the kind of the obejct can be determined and possibly morphologic and dimension differences can be determined.

In figure 4 processes for recognizing shapes, for determining dimensions, the movement, orientation and shape changes, as well as the identification of real objects reproduced by virtual objects with reference to the kind and the task of these real objects are indicated by a subset 214, while the latter is interfaced with a further subset 314 providing morphologic and dimension data typical of real objects that can be compared with the ones determined in images.

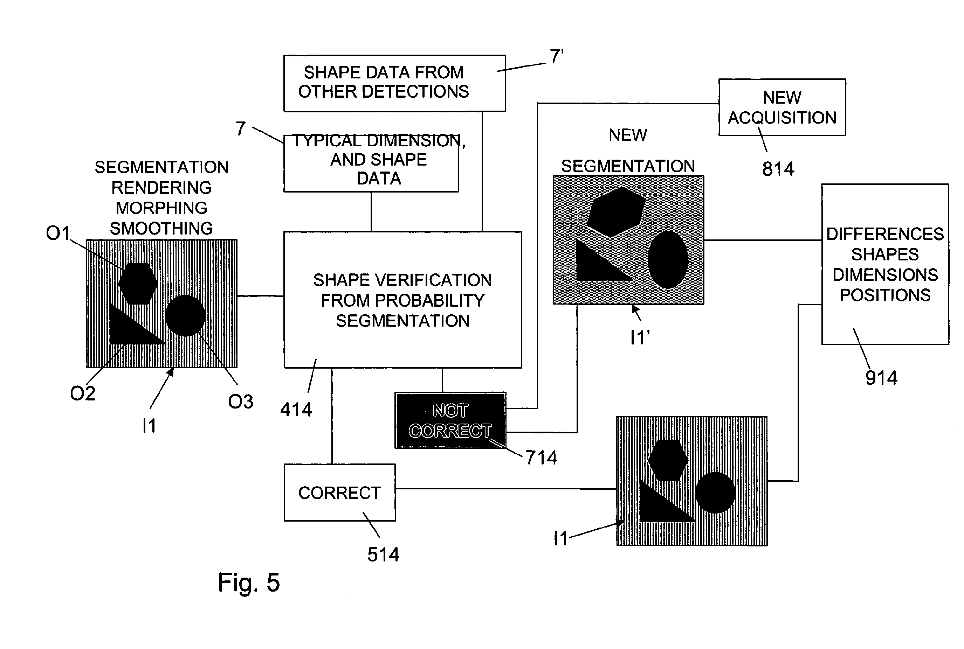

Figure 5 shows a further system for verifying the segmentation and the generation of renderized images as regards the compatibility of the morphology and dimensions of virtual objects identified in images with morphology and/or dimensions typical of corresponding real ones possibly also with reference to morphologic and dimension changes caused by condition changes as in the present case by the presence of a rheumatic disease.

In this case the image segmented and possibly further subjected to reconstruction by rendering possibly in combination with morphing or smoothing treatments, is analysed with reference to the shape and dimensions of objects 01, 02, 03 identified in said image I1 and possibly also of topologic and dimension relations of said objects one with respect to the other in a verification unit 414. To this unit 414 there are provided morphologic and/or dimension data typical of objects considered as to correspond to the ones reproduced in the image I1 and indicated by 01, 02, 03. Such data can be relevant both to an average value and/or to a range included between minimum and maximum values. Moreover typical data can also consider values that are not standard and corresponding to typical pathologic conditions. Processing means can comprise such data inside the database of clinical cases stored for example in the memory or memory area 7 as indicated in figure 1.

Moreover it is possible to make the comparison with morphologic, topologic and dimension data obtained by other measuring or analysing methods, such as by means of other image acquiring means different from the magnetic resonance such as ultrasound, radiologic means etc. said data being also included in the database of clinical cases and being stored in a dedicated memory area indicated by 7' in figure 5.

When the result of the verification system denotes that morphologic and/or dimension and/or topologic data of objects obtained from corresponding virtual objects are compatible with corresponding typical data it is possible to go on as indicated by the box 514 and by the image I1 and in this case for example it is possible to determine shape dimension and position differences of real objects determined by the corresponding virtual objects with respect to corresponding shape, dimension and position data of the same real objects as provided by the database of clinical cases 7 and 7' as indicated by the function box 914. moreover these differences can be used as a standard criterion for defining the existence of a patologic condition considering the rheumatologic point of view and/or for evaluating the evolution degree of the disease if it is present. It is possible to define different numerical comparison parameters according to different comparison functions and constituting numerical values. The comparison of these numerical values of comparison parameters with predetermined threshold values empirically defined on the basis of known clinical cases already allow to define a diagnostic indication.

When the verification unit 414 establishes that there is no compatibility between morphology and/or dimensions and/or position of real objects determined by virtual objects with respect to shape, dimensions and positions of real objects obtained by the database of clinical cases, so image data I1, segmented and/or further renderized and/or possibly subjected also to morphing and/or smoothing are considered as wrong ones 614 and it is possible both to repeat the segmentation and/or rendering process and/or possibly the morphing and/or smoothing process as indicated by the image I1' or even to provide a new acquisition of the image from the body under examination as indicated by 914.

However it is to be noted how all function boxes 4141, 814, 914 are composed of program modules that are executed or can be executed upon call-up from the central processing unit and resident in a memory thereof or can be loaded in said memory.

As regards measures provided to be taken from image data and considered as measures indicating the presence or absence of a rheumatic disease and/or the evolution degree thereof the system according to the invention provides to carry on measures evaluating the inflammatory condition of the synovia and to identify and quantify possible damages to the cartilage or to bony tissue.

Quantitative information highlighting the inflammatory condition, can be obtained in a case by measuring typical parameters of the perfusion of the paramagnetic contrast medium in the synovia. Such method is known and it is described in more details in a previous application to the same applicant.

As an alternative or in combination it is also possible to determine a quantitative measure correlated to the inflammatory condition by the comparative analysis of the contrast of RM images. A particular example is the detection of maps in T1 or T2 obtained at different stages of the disease.

Damages to the cartilage can be quantified by measuring the cartilage, as regards thicknesses or overall volume. From these measures the pathologic condition can be detected, and by repeating at time intervals the examination as well as by comparing results of different examinations, the evolution during the therapy can be detected.

Segmentation tools and rendering tools in combination in turn combined with further morphing and/or smoothing processes allow to create tools segmenting the bony tissue from which a staging of the bony damage can be obtained by the fact that erosions are automatically or semiautomatically highlighted with reference to the number and volume thereof with respect to the volume of the healthy bone.

Finally it is also possible to process images simply by means of an analysis as regards RM contrast by means of which it is possible to automatically highlight pathological regions.

Figure 3 shows all such steps for processing image data. The function block of contrast maps is indicated by 115 with which the synovial inflammatory condition is evaluated as an alternative to the conventional perfusion measure indicated by the function block 116. Function block 117 represents the analysis as regards contrast, that specifically is indicated as a process for processing images of the pixel by pixel or voxel by voxel type and wherein algorithms can be composed of linear or not linear filters such as edge detection filters, or classification algorithms that are linear or not linear such as bayesian networks or artificial neural networks or other algorithms of the evolutive type or combinations of such algorithms.

Each one of these processing tools provides one or more numerical parameters just describing some quantities that are considered to be valid for revealing the presence of the rheumatic disease and/or for determining the evolution condition of the rheumatic disease. They are indicated by P1, P2, P3, P4, P5, P6. It is also possible to combine such parameters with one or more further parameters P7 that have been determined with other kinds of examinations and/or that are about the patient history or personal data and/or pathologic conditions according to previous examinations.

Parameters P1 to P7 are not all necessary and some of them can be omitted or they can be considered as to have a different importance and therefore are evaluated in a different way one with respect to the other.

A mode for determining the presence of the rheumatic disease and/or the evolution stage of such disease according to a very simplified embodiment is the simple comparison for said parameters with threshold numerical values.

In this case each parameter can be individually evaluated or it is possible to consider said parameters as being part of a vector of pahtologic conditions and so to provide an overall evaluation of measured parameters and of threshold ones in the form of a comparison of the length of the corresponding vector whose components are composed of measured parameters P1 to P7 for one of the vectors and of threshold values for said parameters for the comparison one.

Again a variant provides individual parameters to be weighted when their importance is different for determining the diagnostic indication.

For determining the presence of the rheumatic disease and/or the evolution condition thereof it is also possible to use other statistic systems evaluating parameters P1 to P7 or any subcombination thereof.

Finally a particularly developped embodiment provides numerical parameters P1 to P7 to be input values of a classification algorithm or a predictive algorithm such as for example an artificial neural network indicated by 119 in figure 3.

In this case the database of clinical cases and particularly of the specific patient are used for carrying out the training and testing step of the neural network.

It is interesting to consider the fact that the general database can lack in all or some specific data of the patient and so the network can be in an intermediate learning condition, while the learning ends during the step examining the patient by loading the personal clinical database of the patient and by carrying out a further training and testing step by this database. Clinical data of the patient relevant to other preceding examinations both of the same type and of the different type can be stored in a storing movable medium such as a chip-card also known as smart card or the like that is read by the system at the imminence of an examination session by a suitable reader.

Therefore the system according to the present invention have to allow the storing of data obtained by above described methods during different examinations made on each patient.

The personal clinical database of the patient allows also to evaluate in a precise, economic, and rapid way the evolution of the disease both with a theraphy and without it by means of a follow-up both of the simple pathologic condition and possibly or as an alternative of the therapeutic treatment of the rheumatic disease.

Also in this case, the system provides a processing module or tool working on the basis of automatic comparison algorithms that for example are based on "expert systems" or predictive ones and intended to highlight changes occurred between an examination and the other one during the therapeutic treatment.

From the above it is clear that the system according to the present invention provides methods for measuring functional parameters intended to highlight the condition of the rheumatic disease and to allow its follow-up during the theraphy and specifically these methods consist in means for processing images for analysing the synovial condition by highlighting the inflammatory condition, such as the measurement of characteristic parameters of the paramagnetic contrast medium perfusion in the synovia, and/or the comparative analysis of RM image contrast obtained at different stages of the disease. As regards the cartilage the invention provides the segmentation and that is the measure as regards thicknesses or overall volume intended to highlight the pathologic condition and the evolution during the theraphy, as well as the analysis regarding RM contrast aiming at highlighting pathologic regions in the more automatic possible way. At last the invention provides also means for staging the bony damage automatically or semi-automatically highlithing the amount of bony erosions and their volume with respect to the volume of healthy bone.

It is also important to consider the fact that the system according to the present invention allows to deduce diagnosis suppositions or however to provide the medical staff with widening/highlighting cues and not with an automatic definitive diagnosis by the comparison with quantitative parameters measured during the occuring examination.

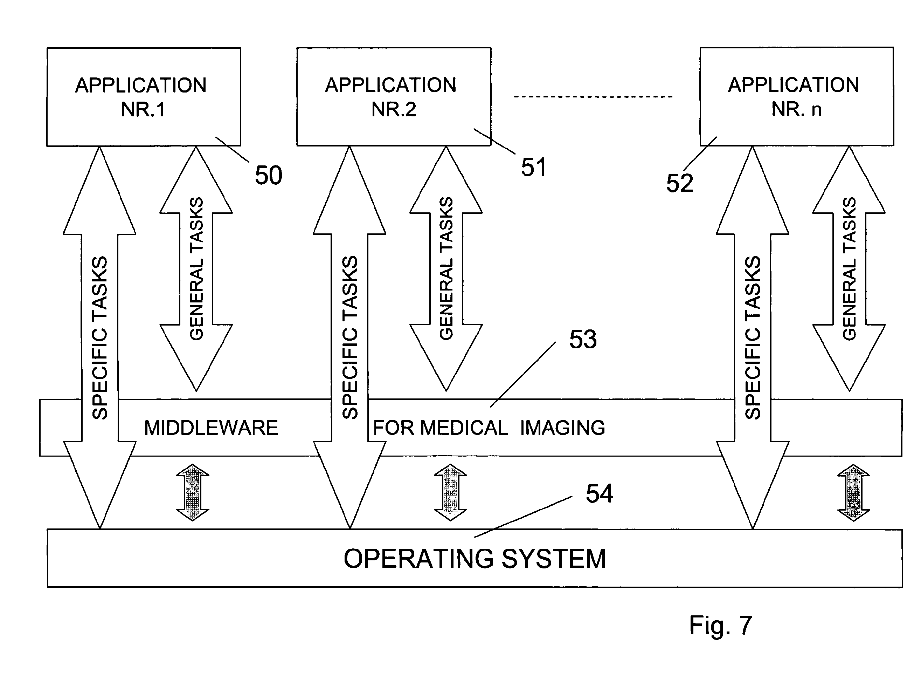

Figure 7 shows a block diagram of the structure of individual application programs constituting software means for processing image data according to different functionalities as segmentation, morphing, modelling etc, which have been listed above.

In this case, various application softwares processing image data are denoted by 50, 51, 52 which are provided in combination with different kinds of image acquiring apparati and/or different kinds of examined or searched diseases and/or different anatomical regions or organs or body parts. Said application softwares comprise routines intended to execute general processing functionalities, such as segmentation, morphing, modelling, registration and other image processing methods already described above or known ones. These type of image data treatments widely leave the specific acquiring mode and other more specific variables out of consideration as regards the type of disease and/or the type of examined organ or anatomical region. However said functionalities require a specific optimization adaptation regarding image acquiring modes, i.e. different image acquiring apparati, diseases and anatomical regions, therefore besides general routines there are provided specific routines such that specific characteristics required by the application software 50, 51, 52 are observed.

As regards the system it is possible to provide a general operating system, such as Windows, or the like that is executed by a computer and a medical imaging and diagnostic program indicated by box 53, which is controlled by the operating system and in turn it comprises different general routines processing image data, and so it provides general processing routines to different applications, whereas the specific application provides further processing routine for the specific disease, the specific image acquiring apparatus or the specific image acquiring technique and/or for the specific anatomical region and/or organ and/or tissue, which specific routines are also controlled by the operating system. Thus, the medical diagnostic and imaging middleware 53 provides general functionalities and it can be in common to all specific applications and each specific application 50, 51, 52 provides to execute only specific routines, the middleware 53 and specific routines being directly controlled both by the operating system 54.

By means of this arrangement, it is possible to generate a single medical and/or diagnostic imaging program, which can be used in any specific combined imaging and/or diagnostic system, considerably limiting routines to be made ad hoc for the specific application.

This allows not only to have a saving in costs, but also to easily provide upgrades for diagnostic helping systems such as the one of the present invention.

Still according to an advantageous characteristic of the invention, means for treating magnetic resonance signals or images i.e. image data that for a part thereof are composed of software programs having CAD functionalities can be resident on the MRI apparatus such as described above or they can be also provided on a separate system. Preferentially, said means can be simultaneously provided on different places such as for example both at the primary physician, the rheumatologist, radiologist, and all this in order to guide the diagnostic/therapeutic procedure. Briefly a part of software providing to constitute treating means having CAD functionalities can be provided and executed both on the image acquiring apparatus and/or on a separate additional console. It is also possible to provide a system having two processing units one of which firmly integrated with the image acquiring apparatus and the other one connected by means of an interface allowing its separation both as regards the functional point of view and the mechanical one. In this case processing means with CAD functionalities can be partly provided both in memories associated to firmly integrated processing means and in memories associated to separable and removable processing means in order to be executed on both said processing means in a independent way. Parts of processing means with CAD functionalities, such as for example parts regarding the interaction with image acquiring hardware or parts regarding the collection of data from other diagnostic apparatus or other sources such as central clinical databases and other units intended to provide data can be stored in memories associated only to one of the two computers correspondingly to the task they have to carry out within the system. Typically the separable and removable computer can be composed of a portable PC, such as a lap top, a notebook a sub notebook, or a PDA or the like having interfaces for the electric and mechanical connection to the MRI apparatus comprising the other computer in the form of an integrated unit also from the physical point of view.

The overall rheumatologic CAD that is the whole suite of programs and routines according to the invention is completed by further data in addition to the ones relevant to images acquired by the specific system such as for example various data about the patient history, laboratory examinations, RX, Echography, etc etc there being possible for the CAD itself to provide to guide the choice on the subsequent diagnostic and therapeutic step for a statistic evaluation of said data. In this case guided choice mechanisms can be based on objective criteria composed of data provided from different analyses and different examinations.

In the case of the removable computer processing means are composed of a mere software that can be executed on any computers and so processing means according to the present invention at least partly are composed of a program that can be executed by a computer and stored on a removable medium as well as by said medium upon which the program is stored.

## Claims

**1.** System for determining diagnostic indications which system comprises:
at least an apparatus for acquiring diagnostic images;
and image processing means for recognizing and measuring qualitative, quantitative, morphologic and/or dynamic characteristics of one or more objects reproduced in acquired images by pixels, or voxels or image data thereof
**characterized in that**
processing means and the apparatus for acquiring diagnostic images being integrated within the same device and the processing of the image or images being carried out directly at the end of the acquisition of the image or images as the final and/or intermediate step of the diagnostic imaging session or process.

**2.** System according to claim 1, **characterized in that** means acquiring image signals and determining image data therefrom, means storing said image data, means generating images from image data and displaying them and means processing digital images comprising at least means determining functional morphologic characteristics of objects reproduced in images from image data and/or means determining qualitative and/or quantitative characteristics of the type of objects reproduced in images from image data and classificating them on the basis of said qualtitative and/or quantitative type characteristics, are integrated in the same device and wherein the system provides as output one or more acquired images or a sequence of acquired images and functional morphologic information and/or information about qualitative and/or quantitative characteristics of the type of objects reproduced in images and about the classification thereof on the basis of said qualitative and/or quantitative type characteristics.

**3.** System according to claims 1 or 2, **characterized in that** image data are directly used in order to obtain said diagnostic indications by their direct processing.

**4.** System according to claims 1 or 2, **characterized in that** image data are subjected to different processing processes each of which is intended to prepare the image or images and/or image data for determining and/or extracting therefrom numerical values of parameters measuring pathologic conditions (absence/presence and/or evolution degree of the disease) or each of which is intended to determine numerical values of parameters measuring pathologic conditions by images or by image data.

**5.** System according to claim 4, **characterized in that** it comprises different chains for determining or extracting different parameters measuring pathologic conditions from image data and/or from images, which chains work in parallel one with the other and each one provides values for one or more numerical parameters measuring the disease in a independent way one with the other.

**6.** System according to claim 3 or 5, **characterized in that** processing processes are carried out by one or more sub-sections and in turn they can be composed of various process steps treating images and/or image data and moreover they can be numerical input data of further processing means working on the basis of classification algorithms and/or predictive algorithms, which provide final indications about the absence/presence of diseases and/or about their evolution degree.

**7.** System according to claim 6, **characterized in that** different image processing processes are used even in a linked way or as a succession.

**8.** System according to one or more of preceding claims, **characterized in that** it provides the extraction of dimension measures, such as length, width, diameter, surface, volume and other dimension measures of objects represented in images.

**9.** System according to claim 8, **characterized in that** it provides to treat image data by means of a segmentation process for identifying subsets of pixels, voxels or image data, which subset elements have such characteristics to be considered as being part of an individual object and by the following univocal and automatic identification of the subset of pixels, voxels or image data with a real object that is present in the area or volume of the body under examination of which the image or images has/have been acquired.

**10.** System according to claim 9, **characterized in that** it provides verification means in order to guarantee that the process segmenting and identifying objects reproduced by subsets provided by the segmentation are correct.

**11.** System according to one or more of preceding claims, **characterized in that** it provides means for treating the image or images and/or image data and a processing by rendering tools.

**12.** System according to claim 11, **characterized in that** it provides means for generating virtual scenarios simulating the reality on the basis of image data.

**13.** System according to one or more of preceding claims, **characterized in that** it comprises means for subjecting images or image data to a morphing (modelling), and/or smoothing, and/or pattern recognition, and/or edge detection, and/or feature tracking, and/or registration treatment by means of which image data are only subjected to a transformation such that they can be subsequently processed by automatic or semiautomatic tools measuring or reading numerical parameters indicating the pathologic condition.

**14.** System according to one or more of preceding claims, **characterized in that** it has means for determining a measure in the form of one or more numerical parameters of the shape of an object reproduced in images and/or of changes to said shape in time both merely regarding the morphologic characteristics and also regarding dynamic characteristics such as elongation and/or compressive stresses and rates at which deformations occur.

**15.** System according to claim 14, **characterized in that** it provides means for processing images to carry out measurements of the time change of the intensity of acquisition signals and/or of the intensity of image pixels or voxels in one or more regions and means for determining changing curves from which specific parameters of functions describing said curves and/or other numerical parameters can be determined such as perfusion curves of contrast media.

**17.** System according to one or more of the preceding claims, **characterized in that** it comprises means for determining qualities or the state of tissues reproduced in the image or images by analysing contrast maps of said images.

**18.** System according to one or more of preceding claims, **characterized in that** determined numerical parameters are input data of processing means working on the basis of classification and/or predictive algorithms, or numerical data of measurement parameters are parameters for determining in an independent way for each processing process an indication about the probability of the presence/absence and/or the evolution degree of the searched disease.

**19.** System according to one or more preceding claims, **characterized in that** it comprises a database of known clinical cases for which clinical cases the condition of the presence/absence and/or the evolution degree of the disease is known and for which clinical cases numerical measurement parameters have been determined which can be defined by various processing processes and means for the comparison between values of measurement parameters obtained for the case under examination and values of the database of known clinical cases.

**20.** System according to claim 19, **characterized in that** it provides comparison means which generate a reference value for each measurement numerical parameter corresponding to a certain function of all values of corresponding parameters of known clinical cases having the same disease and/or the same evolution degree of the disease.

**21.** System according to claim 20, **characterized in that** it has means for the comparison between values of numerical parameters measuring the pathologic condition determined for the case under examination and values of corresponding parameters of clinical cases of the database of known clinical cases that carry out said comparison by using more predictive or statistic type tools such as linear regressions, classification networks and/or clustering tools.

**22.** System according to one or more preceding claims, **characterized in that** means determining results regarding the presence/absence of the disease and/or the evolution degree of it obtained for each different process processing images in turn are input data of processing means working on the basis of predictive and/or classification algorithms and providing the diagnostic indication about the presence/absence and/or the evolution degree of the disease as output.

**23.** System according to one or more preceiding claims, **characterized in that** input data provided to such processing means further comprise general information, such as for example personal data and/or other information about life conditions and uses and/or pathologic information of the patient under examination or about his physiologic parameters.

**24.** System according to one or more of preceding claims, **characterized in that** data are further parameters measuring pathologic conditions computed in previous examinations and/or indications about the presence/absence and/or the evolution degree of the disease provided by classification and/or predictive processing means in previous examinations and/or as an alternative or in combination data relevant to indications about the presence/absence and/or the evolution degree of the disease provided by individual processing processes.

**25.** System according to one or more of preceding claims, **characterized in that** processing means that provide CAD functionalities are composed of so called software "tools", that is programs for treating data loaded in memories of a computer and are executed by it, for example a computer of the personal computer type or the like.

**26.** System according to one or more of preceding claims, **characterized in that** it has a direct feedback and/or interaction communication between said means for acquiring images and said processing means with CAD functionalities, which interaction allows to automatically and/or semi-automatically optimizing settings of acquiring means upon the control of processing means and possibly also viceversa.

**27.** System according to claim 26, **characterized in that** it comprises means for generating acquisition settings controlled by quality verification means associated and/or present in processing means.

**28.** System according to claim 27, **characterized in that** image processing means for recognizing and measuring qualitative, quantitative, morphologic and/or dynamic characteristics of one or more objects reproduced in acquired images and/or of image data and/or of acquisition signals from which image data and said images are extracted have means for analysing one or more qualitative parameters of images and/or of image data and/or acquisition signals, which means generate a command changing settings of acquiring means.

**29.** System according to claims 27 or 28, **characterized in that** the interaction between acquiring means and image processing means is carried out according to iterative steps, i.e. by carrying out different subsequent steps acquiring images and subsequently processing them by processing means with CAD functionalities, as well as qualitatively analysing and determining new acquisition parameters and/or new acquisition sequences and subsequently repeating previous acquisition and processing steps. Said steps can be iteratively repeated up to a certain number of times till processing means reach predetermined minimum threshold values of statistic reliability parameters of results.

**30.** System according to one or more of preceding claims, **characterized in that** it comprises means for storing a database of diagnostic images and of processing data with CAD means relevant to each patient, which patient database possibly comprises further data, such as image acquisition settings, used processing means, patient conditions at each image acquisition defined on the basis of other physic or physiologic parameters.

**31.** System according to claim 30, **characterized in that** data of the database are used to carry out "follow up" analyses consisting in repeating processes acquiring images and processing image data and in comparing them with images and image data processings of the same patient regarding one or more previous acquisitions or processings and/or in comparing them with reference data describing the evolutive condition included in said database.

**32.** System according to one or more of preceding claims, **characterized in that** processing means are composed of one or more computer programs that can be executed by a computer as a PC or the like, whereas the architecture of said means comprises a managing logic or program and a set of application programs each of which is specifically intended to carry out a different process for processing images or image data or a specific control step such as the qualitative verification and the change of acquisition settings, as well as the repetition of the acqusition.

**33.** System according to claim 32, **characterized in that** each application software comprise image processing routines of the general type that are taken or called up by an image data processing program that is managed by the computer oprating system and which routines are independent of the specific image acquisition technique and/or of specific processing modes relevant to the specific disease and/or to specific anatomical region or regions, to specific organs and/or to specific tissues, and image data processing routines that are carried out in order to satisfy processing modes determined by the specific image acquisition technique and/or by specific processing modes relevant to the specific disease and/or to specific anatomical region or regions, to specific organs and/or specific tissues and are directly managed by the computer operating system.

**34.** System according to one or more of preceding claims, **characterized in that** it comprises two separated processing hardware units that can be electrically and/or mechanically connected one with the other a first processing unit being provided with its own memories for the processing programs and being integrated with the image acquiring apparatus whereas a second hardware processing unit is also provided with dedicated memories for the processing programs said second unit being separable from the image acquiring apparatus and from the first processing unit integrated therein, both from the electrical and mechanical point of view and said second processing unit being removable and it can be used separately from the first one and from the image acquiring apparatus.

**35.** System according to claim 34, **characterized in that** at least a part of software programs composing means for treating image data are stored in memories of both processing units and are individually executed by both said units.

**36.** System according to claim 34 or 35, **characterized in that** at least a part of software programs composing means for treating image data are stored in memories of only one of said two processing units and are individually executed by said corresponding processing unit.

**37.** System according to one or more of claims 34 to 36, **characterized in that** it comprises various separated image acquiring units working according to different acquisition techniques, whereas each image acquiring unit has a first hardware unit processing image data integrated therein wherein programs constituting image treating means are loaded or can be executed by it, whereas there is provided at least a second processing hardware unit wherein at least a part of software programs constituting image treating means are loaded and can be executed by it, each one of said different image acquiring unit and corresponding first processing hardware units that can be mechanically and/or electrically and/or functionally connected to said second hardware processing unit for downloading in said second hardware processing unit image data that have been processed and/or partially processed by the corresponding first hardware processing unit and/or that are present in it.

**38.** System according to one or more of the preceding claims, **characterized in that** it comprises movable or portable storing means, such as a chip, an electronic card, a so called memory card, a floppy disk, a portable hard disk and/or a rom or ram DVD or CD that are personal for each patient and upon which data of a database of diagnostic images and processing data with CAD means relevant to a specific patient are stored, which patient database possibly comprises further data, such as image acquisition settings, used processing means, patient conditions at each image acquisition that are defined on the basis of other physic or physiologic parameters, and/or clinical and/or diagnostic results relevant to other diseases and/or obtained by other type of exminations, whereas the system has means for reading/writing data from said personal storing means which reading/writing means are integrtaed with the image acquiring apparatus and/or with a remote unit processing image data.
